# EUROPEAN PATENT APPLICATION

(11) **EP 2 455 048 A1**
(43) Date of publication of application: **23.05.2012**
(21) Application number: 10799798.3
(22) Date of filing: 06.07.2010
(51) Int. Cl.: A61F 13/49, A61F 13/15, A61F 13/53, A61F 13/534

(54) **ABSORBENT BODY AND ABSORBENT ARTICLE**

(30) Priority: 13.07.2009 JP 2009165016
(71) Applicant: Unicharm Corporation, Ehime 799-0111 (JP)
(72) Inventor: NAKASHITA, Masashi, Kanonji-shi Kagawa 769-1602 (JP); KONISHI, Takayoshi, Kanonji-shi Kagawa 769-1602 (JP); MIZUTANI, Satoshi, Kanonji-shi Kagawa 769-1602 (JP)
(74) Representative: Knights, Rupert
(86) International application number: PCT/JP2010/061735
(87) International publication number: WO 2011/007740

(57) **Abstract**

To provide an absorbent core comprising a gelling region and a thickening region, as well as an absorbent article containing the absorbent core.

[SOLUTION MEANS]

Absorbent core 1 formed from at least two layers, **characterized in that** the absorbent core 1 has in upper layer 2 gelling region 4 comprising a gelling agent that contains a polysaccharide capable of thickening in the presence of a polyvalent metal ion and a substance that can supply a polyvalent metal ion, and has in lower layer 3, thickening region 5 comprising a thickening agent.

## Description

### [Technical Field]

The present invention relates to an absorbent core comprising a gelling region and a thickening region, as well as to an absorbent article containing the absorbent core.

### [Background Art]

Absorbent cores, and absorbent articles containing them, are used in disposable diapers, sanitary products, medical blood absorbent articles, pet rearing products and the like for treatment of liquids including body fluids and excreted fluids, and they are required to have excellent water absorption performance. The absorbent cores and absorbent articles containing them must also be lightweight and thin.

Acrylic acid-based absorbers are known as absorbers that can absorb water at several hundred to several thousand times their dry mass, and they are used in a number of products. In particular, absorbent cores wherein super absorbent polymer particles composed of a salt of crosslinked polyacrylic acid are dispersed in pulp fiber are widely used in the fields of disposable diapers, sanitary products, medical blood absorbent articles and pet rearing products.

With acrylic acid-based absorbers, however, the liquid, such as body fluid or excreted fluid is absorbed, held and/or immobilized after reaching the location of the absorber, and time is required until all of the liquid reaches the location of the absorber, such that liquid leakage can occur before immobilization of the liquid. In addition, a relatively large amount of the acrylic acid-based absorber must be used to obtain satisfactory absorption performance, which leads to difficulty in achieving "lighter mass of the absorbent core and absorbent article".

Furthermore, as a property of the acrylic acid-based absorber, its volume increases as the absorber particles incorporate air from the surroundings after liquid absorption, and this tends to increase the thickness after absorption, often leading to "wear discomfort" and/or "reduced absorption performance". Although an acrylic acid-based absorber has a very high absorption rate for ion-exchanged water, its absorption rate for ion-containing liquids, such as body fluids tends to be much lower. Attempts have been made to lower the crosslinking degree of the acrylic acid-based absorber to' increase absorption performance, but this leads to problems, such as reduced gel strength. From the viewpoint of environmental protection, absorbent articles must have water disintegratability and/or biodegradability in some cases, but acrylic acid-based absorbers have not yet reached a satisfactory level of water disintegratability and biodegradability, and therefore a demand exists for new absorbers.

Absorbent cores that do not employ acrylic acid-based absorbers, such as absorbent cores employing polysaccharides, for example, are being studied to improve the various problems associated with acrylic acid-based absorbers. For example, PTL 1 proposes a thickening treatment article for body fluids or excreted fluids, which comprises a polysaccharide that can increase viscosity in the presence of a polyvalent metal ion, wherein the polysaccharide is in a state in which it can dissolve or dissociate in the water of the body fluid or excreted fluid.

### [Citation List]

### [Patent Literature]

[PTL 1] Japanese Unexamined Patent Publication No. 2000-201976

### [Summary of Invention]

### [Technical Problem]

The thickening treatment article for body fluids or excreted fluids disclosed in PTL 1 has presented problems, in that when the polysaccharide is situated in the uppermost layer on the surface (absorbing) side and body fluid is absorbed, the body fluid that has become thickened by the polysaccharide but not yet gelled readily returns to the skin, and the thickened body fluid prevents further absorption, thus tending to result in "skin roughening".

Moreover, the thickening treatment article for body fluids or excreted fluids disclosed in PTL 1 has an acrylic acid-based absorber situated on the lowermost layer, and therefore has the problems regarding "product mass reduction", "wear discomfort" and/or "reduced absorption performance" which are characteristic of acrylic acid-based absorbers.
An absorbent core and absorbent article that can overcome the problems of acrylic acid-based absorbers and polysaccharide-based absorbers have therefore been desired.

### [Solution to Problems]

As a result of much diligent research with the aim of solving the problems described above, the present inventors have found that the aforementioned problems can be solved by an absorbent core formed from at least two layers, **characterized in that** the absorbent core has in an upper layer a gelling region comprising a gelling agent that contains a polysaccharide capable of thickening in the presence of a polyvalent metal ion and a substance that can supply a polyvalent metal ion, and has in one or at least one of a plurality of lower layers, a thickening region comprising a thickening agent, and the invention has been completed upon this finding.

Specifically, the present invention relates to the following aspects.

### [Aspect 1]

An absorbent core formed from at least two layers, **characterized in that** the absorbent core has in an upper layer a gelling region comprising a gelling agent that contains a polysaccharide capable of thickening in the presence of a polyvalent metal ion and a substance that can supply a polyvalent metal ion, and has in one or at least one of a plurality of lower layers, a thickening region comprising a thickening agent.

### [Aspect 2]

The absorbent core according to aspect 1, wherein the polysaccharide capable of thickening in the presence of a polyvalent metal ion is selected from the group consisting of sodium alginate, propyleneglycol alginate, pectin, gellan gum, carrageenan, glucomannan and guar gum.

### [Aspect 3]

The absorbent core according to aspect 1 or 2, wherein the substance that can supply a polyvalent metal ion is selected from the group consisting of calcium phosphate, calcium chloride, calcium lactate, calcium gluconate, calcium acetate, aluminum sulfate, aluminum nitrate, aluminum phosphate and aluminum acetate.

### [Aspect 4]

The absorbent core according to any one of aspects 1-3, wherein the thickening agent is selected from the group consisting of sodium alginate, propyleneglycol alginate, pectin, gellan gum, carrageenan, glucomannan, guar gum, polyethylene oxide and polyethylene glycol.

### [Aspect 5]

The absorbent core according to any one of aspects 1-4, which is composed of two layers, the upper layer and the lower layer.

### [Aspect 6]

The absorbent core according to any one of aspects 1-5, wherein the gelling region further contains an organic acid and/or a polyvalent metal ion scavenger.

### [Aspect 7]

The absorbent core according to aspect 6, wherein the organic acid is selected from the group consisting of glucono-δ-lactone, adipic acid, citric acid, malic acid, tartaric acid, lactic acid and acetic acid.

### [Aspect 8]

The absorbent core according to aspect 6 or 7, wherein the polyvalent metal ion scavenger is selected from the group consisting of sodium citrate, sodium polyphosphate, sodium hexametaphosphate and sodium pyrophosphate.

### [Aspect 9]

The absorbent core according to any one of aspects 1-8, wherein the thickening region contains a polyvalent metal ion scavenger.

### [Aspect 10]

An absorbent article comprising a liquid-permeable top sheet and a liquid-impermeable back sheet, and the absorbent core according to any one of aspects 1-9 situated between the liquid-permeable top sheet and the liquid-impermeable back sheet.

### [Aspect 11]

The absorbent article according to aspect 10, having water disintegratability.

### [Aspect 12]

The absorbent article according to aspect 10 or 11, having biodegradability.

### [Advantageous Effects of Invention]

In the absorbent core of the invention, the gelling region of the upper layer gels after absorbing a large amount of body fluid, and the liquid absorption rate per unit mass of the polysaccharide is increased, thus allowing reduced mass and reduced thickness of the absorbent core.
Also, the gelling region of the upper layer in the absorbent core of the invention can also prevent migration of thickened liquid, and therefore deformation of the absorbent core can be minimized.
Furthermore, since the gelling region of the upper layer in the absorbent core of the invention can also prevent exudation of the thickened liquid, it is possible to prevent leakage of absorbed liquid and skin eruption.

The thickening region in the lower layer of the absorbent core of the invention thickens liquids and retains large amounts thereof in a flow-resistant state, while also increasing the liquid absorption rate per unit mass of the polysaccharide, and this allows reduced mass and reduced thickness of the absorbent core and absorbent article.

### [Brief Description of Drawings]

Fig. 1 is a cross-sectional view of an absorbent core comprising a front sheet, an upper layer, a lower layer and a rear sheet in that order from the top, wherein the upper layer is a gelling region and the lower layer is a thickening region.
Fig. 2 is a cross-sectional view of an absorbent core comprising a front sheet, an upper layer, a lower layer and a rear sheet in that order from the top, wherein the upper layer and the center region of the lower layer are gelling regions, and the peripheral regions of the lower layer are thickening regions.
Fig. 3 is a cross-sectional view of an absorbent core comprising a front sheet, an upper layer, a lower layer and a rear sheet in that order from the top, wherein the upper layer and the peripheral regions of the lower layer are gelling regions, and the center regions of the lower layer are thickening regions.
Fig. 4 is a front-side (absorbing-side) view of a lower layer of which the entirety is a thickening region.
Fig. 5 is a front-side (absorbing-side) view of a lower layer of which the center region in the widthwise direction of the absorbent core is a thickening region.
Fig. 6 is a front-side (absorbing-side) view of a lower layer of which the center region in the lengthwise direction of the absorbent core is a thickening region.
Fig. 7 is a front-side (absorbing-side) view of a lower layer of which the center region in the widthwise direction of the absorbent core and the lengthwise direction of the absorbent core is a thickening region.
Fig. 8 is a cross-sectional view of an absorbent article comprising a liquid-permeable top sheet and a liquid-impermeable back sheet, and the absorbent core shown in Fig. 1 situated between the liquid-permeable top sheet and the liquid-impermeable back sheet.

### [Description of Embodiments]

The present invention will now be explained in greater detail.

### <Absorbent core>

### [Gelling region]

As used herein, "gel" refers to a non-fluid state, and "gelled" refers to a change of an entity from a fluid state to a non-fluid state.
Also as used herein, "gelling region" is a region in which an absorbed liquid is to be gelled.

The gelling region is in the upper layer of the absorbent core, and "upper layer" means the layer which is above the lower layer containing the thickening region. The upper layer may be any layer which is above the lower layer containing the thickening region, and it may even be the uppermost layer in the absorbent core, for example.

### [Polysaccharide capable of thickening in the presence of polyvalent metal ion]

As used herein, "polysaccharide" refers to a saccharide comprising a plurality of bonded monosaccharides.
As used herein, "polysaccharide capable of thickening in the presence of a polyvalent metal ion" refers to a polysaccharide that thickens the system in the presence of a polyvalent metal ion as described below.

Examples of polysaccharides capable of thickening in the presence of polyvalent metal ions including sodium alginate, propyleneglycol alginate, pectin, gellan gum, carrageenan, glucomannan, guar gum, locust bean gum, xanthan gum, glucose, carboxymethyl starch, mannose, galactose, arabinose, fucose, ribose, fructose and dextran. Preferred are sodium alginate, propyleneglycol alginate, pectin, gellan gum, carrageenan, glucomannan and guar gum. Sodium alginate is particularly preferred from the viewpoint of availability.

Sodium alginate is a polysaccharide produced by marine algae, and it forms the major component of sea weed dietary fiber. It dissolves in water and increases in viscosity, and can react with polyvalent metal ions to form a gel. Sodium alginate is a linear polysaccharide composed of two types of uronic acid, β-(1→4)-D-mannuronic acid (M form) and α-(1→4)-L-gluconic acid (G form), and it includes the M form consisting of M-M bonds, the G form consisting G-G bonds, and the random form wherein M and G are randomly arranged, with the nature of the gel differing significantly depending on the proportion of the M and G forms. Because sodium alginate is naturally derived, it is biodegradable and biostable and has properties, such as liquid flow-prevention, adhesion and low abrasiveness, and is used for a wide range of purposes including food additives, adhesives, drugs, cosmetics and wound dressings.

Sodium alginate is marketed in different grades with different viscosities when dissolved, and all such viscosity grades may be used in the absorbent core of the invention. From the viewpoint of easily obtaining a thickening property and/or gel strength, a high viscosity type is preferred, and it may have a viscosity of 100 mPa·s or greater and preferably 500 mPa·s or greater in a 1 mass% aqueous solution.

The amount of the polysaccharide capable of thickening in the presence of a polyvalent metal ion will differ depending on, for example, the type and molecular weight of the polysaccharide, the type and valency of the substance that can supply a polyvalent metal ion, as well as on the purpose of use of the absorbent core and the amount of liquid to be absorbed, but generally it will be 10-1,000 g/m² and preferably 50-500 g/m² as basis weight.

The form of the polysaccharide in the absorbent core of the invention may be, for example, a powder, fibrous structure, film, foam, or a complex immobilized in a base material. The polysaccharide may be used in the absorbent core in any one or more of these forms. For example, two or more forms may be used together by adjusting the timing of sol formation and/or gelling of the polysaccharide.

The powder may be a commercially available granular form. However, when the particle sizes of the commercially available granules are small, for example, the commercially available granules may be granulated using an organic solvent or plasticizer or treated by coating or the like with a surfactant, for example, to prevent aggregation. The organic solvent used for granulation may be a lower alcohol, such as methyl alcohol, ethyl alcohol or propyl alcohol, while glycerin may be mentioned as a plasticizer that can be used for granulation.

For example, granulation can be accomplished in a simple manner by mixing the powder with the organic solvent or plasticizer for dispersion, and then drying it. Because the powder has high affinity for liquids, such as body fluids, it will tend to have undissolved portions when it is used in its original form, and it is therefore useful to perform granulation, to produce a larger particle size and to increase the surface area.

The fibrous structure may be produced by any desired method, such as, for example, spinning and drying to form it.
The film may be obtained by forming a film shape and drying it into a sheet. The film will generally be smooth and have a uniform thickness, but it may also have irregularities or a 3-dimensional structure by embossing or the like. It may also have perforations of different shapes or notches in a discontinuous pattern, such as a zigzag shape, or it may be in the form of fragmented flakes. The film may consist of a monolayer or multiple layers, and the dissolution rate or dissolution volume may vary for each layer.

The foam may be produced by any desired method, and for example, a foaming agent and/or gas and a foam stabilizer may be used to encapsulate the foam in the polysaccharide solution, and formed a film, and dried it to obtain a foam body. The foaming ratio of the foamed body is not particularly restricted, and for example, foaming may be to a several-fold or several dozen-fold ratio.

The complex may be a complex of a polysaccharide and a base material, such as the polysaccharide immobilized in a base material. A binder may be used to immobilize the polysaccharide in the base material. When a binder is used, it is preferably one that does not inhibit dissolution of the polysaccharide when contacted with a liquid, such as a body fluid. As examples for the binder there may be mentioned starch, carboxymethylcellulose, polyvinyl alcohol and other water-soluble polymers, which are used as water-soluble adhesives.

The base material for the complex is not particularly restricted so long as it can hold the polysaccharide, and any desired base material may be used. The base material may be, for example, a film or sheet, or the same with perforations, notches or torn sections, or a fabric, such as a woven fabric, nonwoven fabric, knitted fabric or mesh, and preferably it is one that does not inhibit dissolution of the polysaccharide during contact with a liquid, such as a body fluid.

### [Substance that can supply polyvalent metal ion]

The "substance that can supply a polyvalent metal ion" which is included in the absorbent core of the invention is a water-soluble substance that can release a divalent or greater metal ion, such as a salt containing a divalent or greater metal ion. The polyvalent ion can crosslink the polysaccharide capable of thickening in the presence of a polyvalent metal ion, forming a three-dimensional network structure with the polyvalent metal ion and the polysaccharide capable of thickening in the presence of a polyvalent metal ion, and can gel the system. The bond between the polysaccharide capable of thickening in the presence of a polyvalent metal ion and the substance that can supply a polyvalent metal ion may be an ionic bond, for example.
As examples of divalent or greater ions there may be mentioned calcium ion and aluminum ion.

The substance that can supply a polyvalent metal ion may be, for example, a water-soluble calcium salt or a calcium salt that is water-soluble in the presence of an organic acid, such as calcium phosphates including monobasic calcium phosphate, dibasic calcium phosphate, dibasic calcium phosphate dihydrate and tribasic calcium phosphate, or calcium chloride, calcium lactate, calcium gluconate or calcium acetate, or a water-soluble aluminum salt or an aluminum salt that is water-soluble in the presence of an organic acid, such as aluminum sulfate, aluminum nitrate, aluminum phosphate or aluminum acetate.

For purposes in which a fast system gelling speed is preferred there may be used a substance with high water solubility, and for purposes in which a slow system gelling speed is preferred there may be used a substance with low water solubility or with water-solubility in the presence of an organic acid.

The following is the mechanism by which the substance that can supply a polyvalent metal ion forms a three-dimensional network structure with the polysaccharide capable of thickening in the presence of a polyvalent metal ion.
(1) Each component of the gelling agent begins to dissolve in the liquid that permeates the absorbent core,
(2) dissolution of the polysaccharide capable of thickening in the presence of a polyvalent metal ion results in increased viscosity of the liquid, and inhibition of the flow property (thickening), and
(3) the polyvalent metal ion is released from the substance that can supply a polyvalent metal ion, and the released polyvalent metal ion forms a three-dimensional network structure with the polysaccharide.

The amount of the substance that can supply a polyvalent metal ion will differ depending on, for example, the type and valency of the substance, the type and molecular weight of the polysaccharide, and on the purpose of use of the absorbent core and the type and amount of liquid to be absorbed, but generally it will be' 2-1000 g/m² and preferably 10-500 g/m² as basis weight.

The form of the substance that can supply a polyvalent metal ion, which is contained in the absorbent core of the invention, may be a powder or a complex immobilized in a base material, for example.
The powder may be commercially available granules used directly as a powder. However, when the particle sizes of the commercially available granules are small, for example, the powder may be treated as explained above under "Polysaccharide capable of thickening in the presence of polyvalent metal ion", to prevent aggregation.

By using an organic acid and a polyvalent metal ion scavenger, described below, with the absorbent core of the invention, it is possible to promote and/or inhibit formation of the three-dimensional network structure, as desired. However, by using a method of coating the substance that can supply a polyvalent metal ion with a' surfactant, gelatin, wafer sheet or the like, or covering it with microcapsules or the like, it is possible to control dissolution of the substance that can supply a polyvalent metal ion, and thus control the timing of formation of the three-dimensional network structure.

The complex may be a complex of the substance that can supply a polyvalent metal ion with a base material, such as one wherein the substance that can supply a polyvalent metal ion is immobilized in a base material. A binder may be used to immobilize the substance that can supply a polyvalent metal ion in the base material. When a binder is used, it is preferably one that does not inhibit dissolution of the substance that can supply a polyvalent metal ion when it is contacted with a liquid, such as a body fluid. As examples for the binder there may be mentioned starch, carboxymethylcellulose, polyvinyl alcohol and other water-soluble polymers, which are used as water-soluble adhesives.

The base material is not particularly restricted so long as it can hold the substance that can supply a polyvalent metal ion, and any desired base material may be used. As examples for the base material there may be mentioned fabrics, such as woven fabrics, nonwoven fabrics, films and sheets, and those mentioned above under "Polysaccharide capable of thickening in the presence of polyvalent metal ion" may be used.

The gelling agent in the absorbent core of the invention may further comprise an organic acid and/or a polyvalent metal ion scavenger, as desired.

### [Organic acid]

According to the invention, the organic acid is an optional component of the gelling agent.

As used herein, "organic acid" refers to an organic compound that can lower the pH of the system. As examples of organic acids to be used in the absorbent core of the invention there may be mentioned glucono-δ-lactone, adipic acid, citric acid, malic acid, tartaric acid, lactic acid and acetic acid. The organic acid may be used alone or in combinations of two or more.

The following is the mechanism by which the organic acid promotes formation of a three-dimensional network structure between the polysaccharide capable of thickening in the presence of a polyvalent metal ion, and the polyvalent metal ion.
(1) Each component of the gelling agent begins to dissolve in the liquid that permeates the absorbent core,
(2) dissolution of the polysaccharide capable of thickening in the presence of a polyvalent metal ion results in increased viscosity of the liquid, and inhibition of the flow property, and
(3) the organic acid dissolves, lowering the pH of the liquid, and particularly when glucono-δ-lactone is used as the organic acid, equilibrium reaction takes place with gluconic acid, gradually lowering the pH of the solution, and
(4) lowering of the pH promotes release of the polyvalent metal ion from the substance that can supply a polyvalent metal ion, thus promoting formation of a three-dimensional network structure between the released polyvalent metal ion and the polysaccharide capable of thickening in the presence of a polyvalent metal ion.

The amount of organic acid is not particularly restricted and will differ depending on, for example, the types and amounts of the polysaccharide capable of thickening in the presence of a polyvalent metal ion, the substance that can supply a polyvalent metal ion and the polyvalent metal ion scavenger, but it will generally be an amount that can lower the pH of the system, adjust the dissolution rate of the substance that can supply a polyvalent metal ion, and promote formation of a three-dimensional network structure, and it may be determined as appropriate.

The form of the organic acid, which is contained in the absorbent core of the invention, may be a powder or a complex immobilized in a base material, for example.
The powder may be commercially available granules used directly as a powder. However, when the particle sizes of the commercially available granules are small, for example, the powder may be treated as explained above under "Polysaccharide capable of thickening in the presence of polyvalent metal ion", to prevent aggregation.

The complex may be a complex of an organic acid and a base material, such as the organic acid polysaccharide immobilized in a base material. A binder may be used to immobilize the organic acid in the base material. When a binder is used, it is preferably one that does not inhibit dissolution of the organic acid when contacted with a liquid, such as a body fluid. As examples for the binder there may be mentioned starch, carboxymethylcellulose, polyvinyl alcohol and other water-soluble polymers, which are used as water-soluble adhesives.

The base material is not particularly restricted so long as it can hold the organic acid, and any desired base material may be used. As examples for the base material there may be mentioned fabrics, such as woven fabrics, nonwoven fabrics, films and sheets, and those mentioned above under "Polysaccharide capable of thickening in the presence of polyvalent metal ion" may be used.

When an organic acid is added to the gelling region, formation of the three-dimensional network structure is promoted, and as a result gelling of the gelling region is accelerated and return to the skin can be controlled in a simple manner, thus simplifying the structure of the absorbent core.

### [Polyvalent metal ion scavenger]

According to the invention, the polyvalent metal ion scavenger is an optional component of the gelling agent.
As used herein, "polyvalent metal ion scavenger" refers to, for example, a compound that scavenges the substance that can supply a polyvalent metal ion, or a polyvalent metal ion from a liquid, such as a body fluid, or for example, a compound that forms a complex with the polyvalent metal ion. The polyvalent metal ion scavenger used in the absorbent core of the invention may be, for example, a carboxylic acid salt, such as sodium citrate or a phosphoric acid salt, such as sodium polyphosphate, sodium hexametaphosphate or sodium pyrophosphate.

The following is the mechanism by which the polyvalent metal ion scavenger inhibits formation of a three-dimensional network structure between the polysaccharide capable of thickening in the presence of a polyvalent metal ion and the substance that can supply a polyvalent metal ion.
(1) Each component of the gelling agent begins to dissolve in the liquid that permeates the absorbent core,
(2) dissolution of the polysaccharide capable of thickening in the presence of a polyvalent metal ion results in increased viscosity of the liquid, and inhibition of the flow property (thickening),
(3) the dissolved polyvalent metal ion scavenger sequesters the polyvalent metal ion released from the substance that can supply a polyvalent metal ion (forming a complex with the polyvalent metal ion, for example), and
(4) sequestering of the polyvalent metal ion prevents the polyvalent metal ion concentration in the solution from rising, and inhibits formation of the three-dimensional network structure.

The amount of the polyvalent metal ion scavenger is not particularly restricted, and it will differ depending on the type and amount of the polysaccharide capable of thickening in the presence of a polyvalent metal ion, the substance that can supply a polyvalent metal ion and the organic acid that are used.

The form of the polyvalent metal ion scavenger, which is contained in the absorbent core of the invention, may be a powder or a complex immobilized in a base material, for example.
The powder may be commercially available granules used directly as a powder. However, when the particle sizes of the commercially available granules are small, for example, the powder may be treated as explained above under "Polysaccharide capable of thickening in the presence of polyvalent metal ion", to prevent aggregation.

The complex may be a complex of the polyvalent metal ion scavenger with a base material, such as one wherein the polyvalent metal ion scavenger is immobilized in a base material. A binder may be used to immobilize the polyvalent metal ion scavenger in the base material. When a binder is used, it is preferably one that does not inhibit dissolution of the polyvalent metal ion scavenger when it is contacted with a liquid, such as a body fluid. As examples for the binder there may be mentioned starch, carboxymethylcellulose, polyvinyl alcohol and other water-soluble polymers, which are used as water-soluble adhesives.

The base material is not particularly restricted so long as it can hold the polyvalent metal ion scavenger, and any desired base material may be used. As examples for the base material there may be mentioned fabrics, such as woven fabrics, nonwoven fabrics, films and sheets, and those mentioned above under "Polysaccharide capable of thickening in the presence of polyvalent metal ion" may be used.

When a polyvalent metal ion scavenger is added to the gelling region, gelling is delayed and the liquid absorption rate per unit mass of the polysaccharide is increased, thus allowing the structure of the absorbent core to be simplified.

### [Gelling agent]

As used herein, "gelling agent" refers to an agent that contains a polysaccharide capable of thickening in the presence of a polyvalent metal ion, a substance that can supply a polyvalent metal ion, and if desired an organic acid and a polyvalent metal ion scavenger, and that gels a liquid, such as a body fluid.

Each component of the gelling agent in the absorbent core of the invention, i.e., the polysaccharide capable, of thickening in the presence of a polyvalent metal ion, the substance that can supply a polyvalent metal ion, and if desired an organic acid and a polyvalent metal ion scavenger, may be separately introduced into the absorbent core. When the components are separately introduced, each component may be introduced in their respective forms described above.

The polysaccharide capable of thickening in the presence of a polyvalent metal ion, the substance that can supply a polyvalent metal ion, and if desired an organic acid and a polyvalent metal ion scavenger, may also be mixed to prepare a gelling agent, and then introduced into the absorbent core. The form of the gelling agent that is introduced, when a gelling agent is prepared beforehand, may be, for example, a powder, fibrous structure, film, foam, or a complex immobilized in a base material.

The powder, fibrous structure, film, foam or complex immobilized in a base material may be in the same form as explained above under "Polysaccharide capable of thickening in the presence of polyvalent metal ion".

The gelling agent used for the invention has low volume swelling during absorption of liquids and low deformation of absorbent articles after absorption, compared to an acrylic acid-based absorber, and therefore' inhibits reduction in absorption performance and alleviates discomfort during wear.
In addition, the gelling agent used for the invention has low volume swelling during absorption of liquids and also low increase in absorbent article thickness after absorption, compared to an acrylic acid-based absorber, and thus inhibits reduction in absorption performance and alleviates discomfort during wear.

### [Thickening region]

As used herein "thickened" refers to a state of higher viscosity than the original viscosity, while "thickening" refers to increasing the viscosity of an object above its original viscosity such that the flow property is lowered in a range wherein the flow property is still maintained.
Also as used herein, "thickening region" is a region in which an absorbed liquid is to be thickened.

The thickening region is in the lower layer of the absorbent core, and "lower layer" means the layer which is below the upper layer containing the gelling region. The lower layer may be any layer which is below the upper layer containing the gelling region, and it may even be the lowermost layer in the absorbent core.

### [Thickening agent]

As used herein "thickening agent" refers to an agent that thickens a liquid, such as a body fluid.
Examples of thickening agents include those mentioned above under "Polysaccharide capable of thickening in the presence of polyvalent metal ion". As such thickening agents there may be mentioned water-soluble polyalkylene oxides, such as polyethylene oxide, and water-soluble polyalkylene glycols, such as polyethylene glycol and polyvinyl alcohol.

The thickening agent may further contain, if desired, gelling components, such as the gelling agent, in a range that does not eliminate the flow property, for the purpose of further increasing the viscosity of the absorbed liquid and preventing leakage from the absorbent core and absorbent article.

On the other hand, a polyvalent metal ion scavenger may be added to the thickening agent to actively prevent gelling. As polyvalent metal ion scavengers there may be used any of the same polyvalent metal ion scavengers mentioned above under "Gelling region".

### [Absorbent core]

As used herein, "absorbent core" refers to products that serve mainly for absorption of liquids and which are used for purposes that include sanitary products such as sanitary napkins and panty liners, sanitary materials, such as disposable diapers, urine leakage-preventing sheets, urine-absorbing pads for incontinent patients, body fluid/blood-absorbing medical goods, wound-dressing materials, cosmetic pack materials, animal excrement-treating materials, agricultural and gardening products, freshness-keeping materials for foods, moisture condensation-proof materials and products to be used in locations that require moisture absorption and/or moisture retention.

The absorbent core of the invention may include a gelling region, thickening region and base material in the absorbent core, a front sheet on the front side (absorbing side), an interlayer sheet and a rear sheet on the rear side.
The base material in the absorbent core may be, for example, a fiber or nonwoven fabric. The base material preferably has water disintegratability and/or biodegradability.

As examples of fiber materials in the absorbent core of the invention there may be mentioned those formed of synthetic fibers, such as polyester fiber, polyacrylonitrile fiber, polyvinyl chloride fiber, polyethylene fiber, polypropylene fiber, polystyrene fiber, polyethylene terephthalate fiber, polyethylene/polypropylene fiber, polyethylene/polyethylene terephthalate composite fiber and polyvinyl alcohol fiber, semisynthetic fibers, such as cellulose-based fiber and protein-based fiber, natural fibers, regenerated fibers, such as cellulose-based fiber including pulp fiber, and rayon fiber, acetate fiber and the like. The fiber may consist of staple fibers or filaments, and may have any desired diameter. The fiber may also be, for example, core-sheath composite fiber, parallel composite fiber, heterogeneous cross-section hollow fiber, microporous fiber, conjugated composite fiber or the like.

The nonwoven fabric used in the absorbent core of the invention, or in the front sheet or interlayer sheet, may be a known nonwoven fabric, such as, for example, a thermal bonded nonwoven fabric, a melt-blown nonwoven fabric, a spunbond/melt-blown/spunbond nonwoven fabric, a spunlace nonwoven fabric, an airlaid nonwoven fabric, a through-air nonwoven fabric (TA) or a point bond nonwoven fabric (PB). A water disintegratable tissue may also be used.

The nonwoven fabric may also have an elastic property. As elastic nonwoven fabrics there may be mentioned nonwoven fabrics produced by meltblown or spunbond methods. The elastic nonwoven fabric may be produced from elastic fibers obtained by melting and spinning a thermoplastic elastomer resin.

The rear sheet of the absorbent core of the invention may be one produced from a polyester, polyacrylonitrile, polyvinyl chloride, polyethylene, polypropylene, polystyrene, polyethylene terephthalate or polyvinyl alcohol, for example. There may also be mentioned air-permeable films, non-air-permeable films, porous films and the like made from high-density polyethylene/low-density polyethylene.

The mechanism by which the absorbent core of the invention absorbs liquids is as follows.
(1) Liquid that has penetrated the surface material of the absorbent core, such as a nonwoven fabric, permeates into the upper layer while most of it migrates into the lower layer,
(2) the thickening agent in the thickening region of the lower layer dissolves in the liquid, and the viscosity of the liquid increases, thus lowering the flow property (thickening),
(3) simultaneously with thickening of the thickening region in the lower layer, the polysaccharide capable of thickening in the presence of a polyvalent metal ion and the substance that can supply a polyvalent metal ion, in the upper layer, dissolve in the liquid and the viscosity of the liquid increases, thus lowering the flow property,
(4) in the upper layer, the dissolved substance that can supply a polyvalent metal ion reacts with the polysaccharide capable of thickening in the presence of a polyvalent metal ion, forming a three-dimensional network structure and causing gelling of the system,
(5) gelling of the upper layer inhibits return of liquid from the lower layer side, and
(6) when repeated liquid absorption occurs, the thickening region in the lower layer reincorporates the liquid and thickens.

When the absorbent core of the invention further contains an organic acid and/or polyvalent metal ion scavenger in addition to the polysaccharide capable of thickening in the presence of a polyvalent metal ion and the substance that can supply a polyvalent metal ion, the mechanism by which the absorbent core absorbs liquid is as follows.
(1) Liquid that has penetrated the surface material of the absorbent core, such as a nonwoven fabric, permeates into the upper layer while most of it migrates into the lower layer,
(2) the thickening agent in the thickening region of the lower layer dissolves in the liquid, and the viscosity of the liquid increases, thus lowering the flow property (thickening),
(3) simultaneously with thickening of the thickening region in the lower layer, the polysaccharide capable of thickening in the presence of a polyvalent metal ion dissolves in the upper layer, thus increasing the viscosity of the liquid and inhibiting the flow property (thickening),
(4-1) when the gelling region contains an organic acid, the organic acid dissolves and lowers the pH of the liquid, and especially when glucono-δ-lactone is used as the organic acid, equilibrium reaction takes place with gluconic acid, thus gradually lowering the pH of the solution,
(4-2) the lowered pH promotes release of the polyvalent metal ion from the substance that can supply a polyvalent metal ion, and formation of a three-dimensional network structure between the released polyvalent metal ion and the polysaccharide capable of thickening in the presence of a polyvalent metal ion,
(5-1) when the gelling region contains a polyvalent metal ion scavenger, the polyvalent metal ion scavenger dissolves in the liquid, sequestering the polyvalent metal ion released from the substance that can supply a polyvalent metal ion (for example, forming a complex with the polyvalent metal ion),
(5-2) sequestering of the polyvalent metal ion prevents the polyvalent metal ion concentration in the solution from rising, and inhibits formation of a three-dimensional network structure,
(6) gelling of the upper layer inhibits return of liquid from the lower layer side, and
(7) when repeated liquid absorption occurs, the thickening region in the lower layer reincorporates the liquid and thickens.

The absorbent core of the invention may further include various additives including plasticizers, aromas, deodorants, inorganic powders, pigments, dyes, antimicrobial materials, pressure-sensitive adhesives and the like, which are commonly used in the art. As examples of plasticizers there may be mentioned glycerin, sorbitol, lactitol, maltitol, erythritol and pentaerythritol. Trehalose may also be used to prevent cracks of the film or sheet during drying. Such additives can impart various functions to the absorber. As examples of inorganic powders there may be mentioned substances that are inert with respect to the liquid, such as silicon dioxide, zeolite, kaolin and clay.

The additives mentioned above may be added to the absorbent core of the invention by methods commonly used in the art.

The structure of the absorbent core of the invention is not particularly restricted so long as it is formed from at least 2 layers, it has a gelling region containing the gelling agent in the upper layer, and it has a structure comprising a thickening region containing a thickening agent in at least one layer of one or a plurality of lower layers, and any such structure may be adopted.

The absorbent core of the invention may further include a super absorbent polymer (SAP) or pulp, in a range that does not interfere with the effect of the invention.
For further improvement in the absorption ability of the absorbent core of the invention, a super absorbent polymer may be further included in the gelling region. As super absorbent polymers there may be mentioned starch-based, cellulose-based, polyacrylic acid-based, polyvinyl alcohol-based, polyacrylamide-based and polyoxyethylene-based polymers, with polyacrylic acid-based polymers being preferred, and sodium polyacrylate-based absorbers being preferred among them.

The construction of the absorbent core of the invention will now be explained with reference to the accompanying drawings. It should be noted that the aspects depicted in the drawings are examples, and do not restrict the invention in any way.

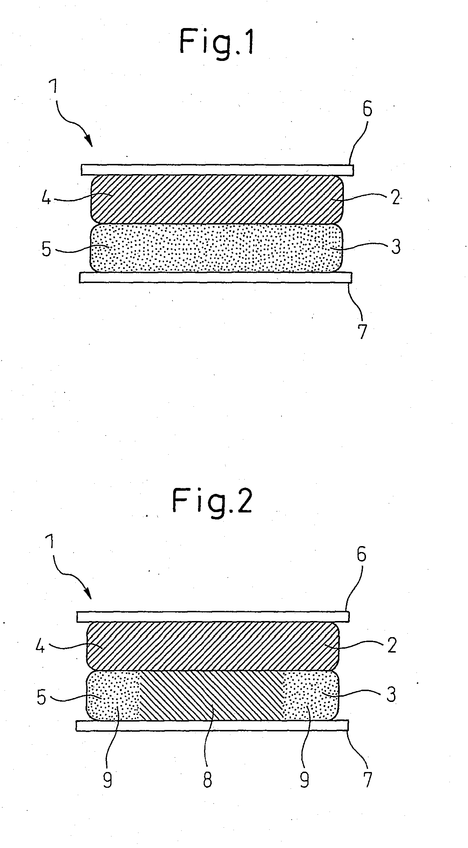

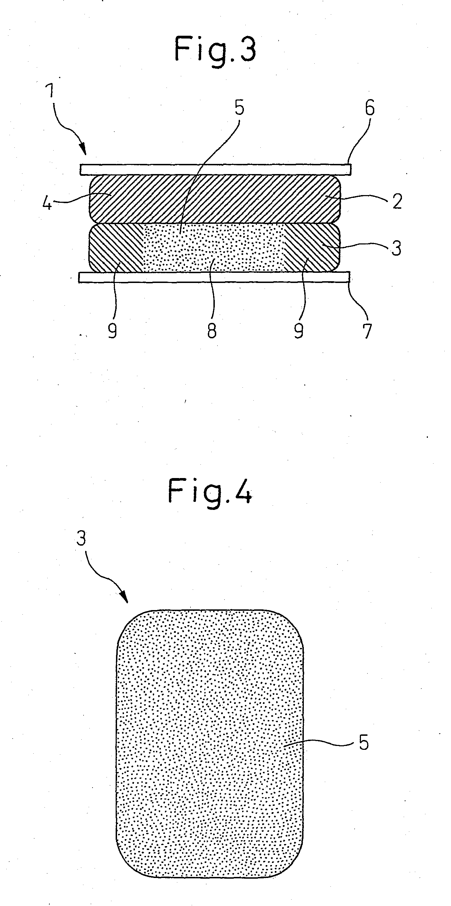

Figs. 1 to 3 are cross-sectional views of absorbent core 1, in which the top side is the front side (absorbing side), i.e. the side that contacts with a human. Figs. 4 to 7 are drawings of lower layer 3 as viewed from the front side (absorbing side).

Fig. 1 is a cross-sectional view of absorbent core 1 comprising front sheet 6, upper layer 2, lower layer 3 and rear sheet 7 in that order from the top, wherein upper layer 2 is gelling region 4 and lower layer 3 is thickening region 5. By providing the construction shown in Fig. 1 it is possible to effectively prevent return of liquid on the front side. The layers in absorbent core 1 may be 3 or more layers, and either or both the interlayer and lower layer may be a thickening region.

Fig. 2 is a cross-sectional view of absorbent core 1 comprising front sheet 6, upper layer 2, lower layer 3 and rear sheet 7 in that order from the top, wherein upper layer 2 and center region of the lower layer 8 are gelling regions 4, and peripheral regions of the lower layer 9 are thickening regions 5. By providing this construction, it is possible to effectively prevent return of liquid on the front side and especially to effectively prevent return of liquid near urinating locations. The center region is the center region in the widthwise direction of the absorbent core and/or the lengthwise direction of the absorbent core.

Fig. 3 is a cross-sectional view of absorbent core 1 comprising front sheet 6, upper layer 2, lower layer 3 and rear sheet 7 in that order from the top, wherein upper layer 2 and peripheral regions of the lower layer 9 are gelling regions 4, and center region of the lower layer 8 is thickening region 5. By providing this construction, it is possible to effectively prevent return of liquid on the front side and especially to effectively prevent return of liquid near perimeter sections.

Fig. 4 is a drawing of lower layer 3 of which the entirety is thickening region 5, as viewed from the front side (absorbing side).

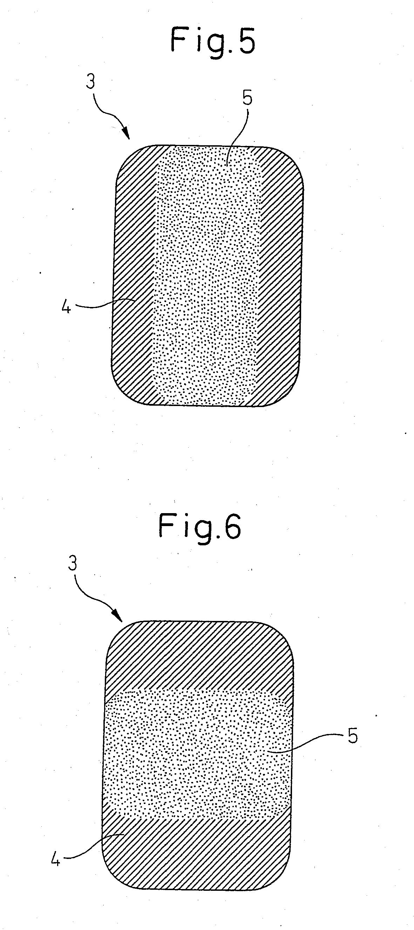

Fig. 5 is a drawing of lower layer 3 of which the center region in the widthwise direction of the absorbent core is thickening region 5, as viewed from the front side (absorbing side). The widthwise direction is the shortest widthwise direction of the absorbent core. In Fig. 5, the peripheral region in the widthwise direction of the absorbent core is the gelling region, but it may also be a region other than the gelling region, such as a pulp region, for example.

Fig. 6 is a drawing of lower layer 3 of which the center region in the lengthwise direction of the absorbent core is thickening region 5, as viewed from the front side (absorbing side). The lengthwise direction is the longest lengthwise direction of the absorbent core. In Fig. 6, the peripheral region in the lengthwise direction of the absorbent core is the gelling region, but it may also be a region other than the gelling region, such as a pulp region, for example.

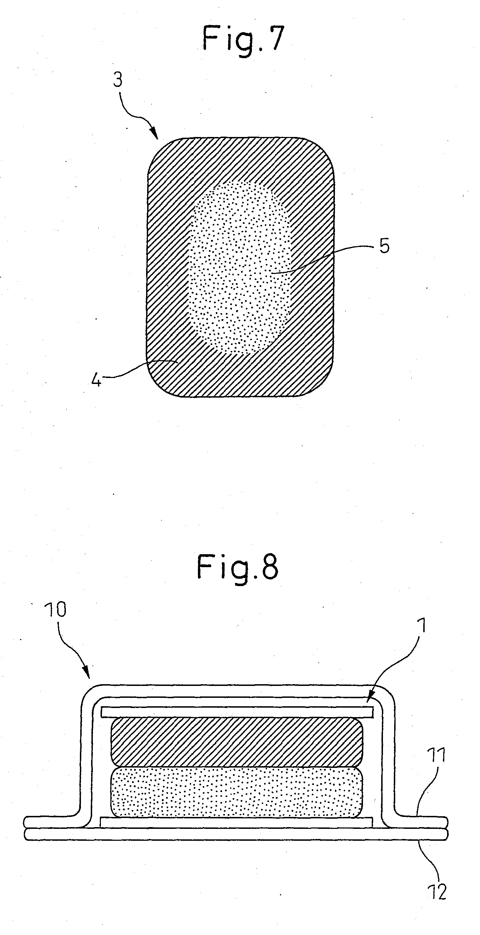

Fig. 7 is a drawing of lower layer 3 of which the center region in the widthwise direction of the absorbent core and the lengthwise direction of the absorbent core is thickening region 5, as viewed from the front side (absorbing side). In Fig. 7, the peripheral region is the gelling region, but it may also be a region other than the gelling region, such as a pulp region, for example.

Since the gelling agent and thickening agent of the absorbent core of the invention have water disintegratability, the front sheet and rear sheet may be selected from among water disintegratable materials to impart water disintegratability to the absorbent core.
In addition, since the gelling agent and thickening agent of the absorbent core of the invention are biodegradable, the front sheet and rear sheet may be selected from among biodegradable materials to impart a biodegradable property to the absorbent core.

### <Absorbent article>

As used herein, "absorbent article" refers to sanitary products, such as sanitary napkins and panty liners, sanitary materials, such as disposable diapers, urine leakage-preventing sheets, urine-absorbing pads for incontinent patients, body fluid/blood-absorbing medical goods, wound-dressing materials, cosmetic pack materials, animal excrement-treating materials, agricultural and gardening products, freshness-keeping materials for foods, moisture condensation-proof materials and articles to be used in locations that require moisture absorption and/or moisture retention.

As used herein, "liquid-permeable top sheet" refers to a sheet situated on the top side (absorbing side), which allows permeation of liquids and particularly body fluids. The liquid-permeable top sheet used in the absorbent article of the invention is not particularly restricted as long as it is a sheet which is permeated by liquids, and as examples there may be mentioned hydrophilic fiber nonwoven fabrics, hydrophobic fiber nonwoven fabrics with multiple openings, and plastic films with openings.

As used herein, "liquid-impermeable back sheet" refers to a sheet situated on the back side (clothing side), which does not allow permeation of liquids and particularly body fluids, under ordinary conditions of use. The liquid-impermeable back sheet used in the absorbent article of the invention is not particularly restricted as long as it is a sheet that is not permeated by liquids, and it may be any sheet used in the art, such as a film or nonwoven fabric.

The structure of the absorbent article of the invention is not particularly restricted so long as the absorbent core can effectively absorb liquids, and particularly body fluids or liquid excreted fluids, and it may have any desired form used in the art, such as the following aspects, for example.
(a) An absorbent article comprising a liquid-permeable top sheet and a liquid-impermeable back sheet, and an absorbent core situated between the liquid-permeable top sheet and the liquid-impermeable back sheet.

(b) An absorbent article comprising a liquid-permeable top sheet and a liquid-impermeable back sheet, and an absorbent core situated between the liquid-permeable top sheet and the liquid-impermeable back sheet, wherein a material that aids diffusion and/or absorption is further situated between the front sheet and the absorbent core and/or between the absorbent core and the rear sheet.
(c) An absorbent article according to (a) or (b) above, wherein either or both the liquid-permeable top sheet and liquid-impermeable back sheet have water disintegratability.
(d) An absorbent article according to any one of (a) to (c) above, wherein either or both the liquid-permeable top sheet and liquid-impermeable back sheet are biodegradable.

Pulp may be mentioned as an example of a material that aids diffusion and/or absorption.

The construction of the absorbent article of the invention will now be explained with reference to the accompanying drawings. It should be noted that the aspects depicted in the drawings are examples, and do not restrict the invention in any way.
Fig. 8 is a cross-sectional view of absorbent article 10 comprising liquid-permeable top sheet 11 and liquid-impermeable back sheet 12, and absorbent core 1 shown in Fig. 1 situated between liquid-permeable top sheet 11 and liquid-impermeable back sheet 12.

### [Examples]

The present invention will now be explained in detail through the following examples, with the understanding that these examples are not limitative of the invention.

### [Production Example 1]

### - Upper layer member -

Materials composed of sodium alginate (B-S, by Kimica Corp.) and dibasic calcium phosphate dihydrate (Wako Pure Chemical Industries, Ltd.) were mixed to uniformity in a mass ratio of 1:1, to obtain a gelling agent. The gelling agent was immobilized over the entire surface of an air-through nonwoven fabric (PE/PP core-sheath composite fiber, basis weight: 40 g/m²) by spraying water with a sprayer, to form an upper layer member with a gelling agent basis weight of 160 g/m².

### - Lower layer member -

A material composed of sodium alginate (B-S, by Kimica Corp.), as a thickening agent, was immobilized over the entire surface of an air-through nonwoven fabric (PE/PP core-sheath composite fiber, basis weight: 40 g/m²) by spraying water with a sprayer, to form a lower layer member with a thickening agent basis weight of 160 g/m².

### - Production of absorbent core 1 -

Absorbent core 1 was produced by situating a rear sheet (polyethylene film), the previously formed lower layer member, an interlayer sheet (tissue, basis weight: 17 g/m²), the previously formed upper layer member and a front sheet (through-air nonwoven fabric, PE/PP core-sheath composite fiber, basis weight: 25 g/m²) in that order, and cutting it to a size of 10 cm × 10 cm.

### [Production Example 2]

Absorbent core 2 was produced according to Production Example 1, except that the material of the upper layer member was changed to a mixture of sodium alginate (B-S, by Kimica Corp.), dibasic calcium phosphate dihydrate (Wako Pure Chemical Industries, Ltd.) and glucono-δ-lactone (Wako Pure Chemical Industries, Ltd.), mixed at a mass ratio of 1:1:1.

### [Comparative Production Example 1]

An acrylic acid-based absorber (San-Dia Polymers, Ltd.) was dispersed on pulp (NB416 by Weyerhaeuser, basis weight: 200 g/m²) to form an acrylic acid-based absorber member with an acrylic acid-based absorber basis weight of 200 g/m²_{.}
Absorbent core 3 was produced by situating a rear sheet (polyolefin film), the member and a front sheet (through-air nonwoven fabric, PE/PP core-sheath composite fiber, basis weight: 25 g/m²) in that order, and cutting it to a size of 10 cm × 10 cm.

### [Comparative Production Example 2]

Absorbent core 4 was produced according to Production Example 1, except that the material of the upper layer member was changed to sodium alginate (B-S, by Kimica Corp.) as a thickening agent, and the material of the lower layer member was changed to an acrylic acid-based absorber (San-Dia Polymers, Ltd.), and it was dispersed on a base material.

### [Comparative Production Example 3]

Absorbent core 5 was produced according to Production Example 1, except that the material of the upper layer member was changed to sodium alginate (B-S, by Kimica Corp.) as a thickening agent.

### [Reference Production Example 1]

Absorbent core 6 was produced according to Production Example 2, except that the material of the lower layer member was changed to a mixture of sodium alginate (B-S, by Kimica Corp.), dibasic calcium phosphate dihydrate (Wako Pure Chemical Industries, Ltd.) and glucono-δ-lactone (Wako Pure Chemical Industries, Ltd.), mixed at a mass ratio of 1:1:1.

### [Examples 1 and 2, Comparative Examples 1-3 and Reference Example 1]

The performances of absorbent cores 1-6 produced in Production Examples 1 and 2, Comparative Production Examples 1-3 and Reference Production Example 1 were evaluated. The procedure was as follows.
1) Each absorbent core is placed in a plastic dish (10 cm × 10 cm × 1 cm), and the thickness is measured.
2) A 40 g portion of artificial urine (prepared by dissolving 2% urea, 0.8% sodium chloride, 0.08% magnesium sulfate heptahydrate and 0.03% calcium chloride dihydrate in ion-exchanged water) is evenly poured into the upper layer member, with the front sheet released, and allowed to stand for 5 minutes.

3) The front sheet is returned to the absorbent core, filter paper (30 g) is placed thereover, and a·3.5 kg weight is set thereon.
4) After 3 minutes, the filter paper and weight are removed and the absorbed dose (Rewet 1) of the filter paper is calculated from the change in weight of the filter paper.
5) Exudation of the gelling agent or thickening agent and the absorbent core form retention are visually evaluated.
6) The front sheet is released and an additional 20 g of artificial urine is evenly poured into the upper layer member and allowed to stand for 5 minutes.

7) The front sheet is returned to the absorbent core, new filter paper (30 g) is placed thereover, and a 3.5 kg weight is set thereon.
8) After 3 minutes, the filter paper and weight are removed and the absorbed dose (Rewet 2) of the filter paper is calculated from the change in weight of the filter paper.
9) Exudation of the gelling agent or thickening agent and the absorbent core form retention are visually evaluated, and the thickness of the absorbent core is also measured.

The form retention was judged on the following scale.
G: Virtually or almost no change in form.
F: Partial change in form.
P: Change in form throughout, inability of absorbent core to maintain form.

The results are shown in Table 1.

[Table 1]

**Table 1. Evaluation results for absorbent articles**

| | | Example 1 | Example 2 | Comp. Ex. 1 | Comp. Ex. 2 | Comp. Ex. 3 | Reference Example 1 |
|---|---|---|---|---|---|---|---|
| | NO. | 1 | 2 | 3 | 4 | 5 | 6 |
| Absorbent core | Upper layer | Gelling agent | Gelling agent | Acrylic acid-based absorber + pulp | Thickening agent | Thickening agent | Gelling agent |
| | Lower layer | Thickening agent | Thickening agent | | Acrylic acid-based absorber | Thickening agent | Gelling agent |
| Rewet 1 (G) | | 1.6 | 1.1 | 0.2 | 0.5 | 1.4 | 7.2 |
| Rewet 2 (G) | | 12.5 | 9.3 | 8.2 | 5.1 | 13.0 | 16.5 |
| Exudation | | No | No | No | Yes | Yes | No |
| Form retention | | G | G | F | P | G | G |
| Initial thickness (MM) | | 5.8 | 5.6 | 3.2 | 5.8 | 5.4 | 5.8 |
| Thickness after absorption (MM) | | 5.8 | 5.7 | 11.2 | 15.8 | 5.7 | 5.8 |

The absorbent cores of Example 1 and Example 2 exhibited absorption (low rewet) equivalent to that of the absorbent core of Comparative Example 1 (conventional acrylic acid-based absorber), while also having more excellent form retention before and after absorption, and more excellent thickness retention before and after absorption. In particular, the absorbent core of Comparative Example 1 exhibited an approximate 3.6-fold increase in volume after absorption of artificial urine twice, while the absorbent cores of Example 1 and Example 2 had virtually no change in volume after absorpti-on of approximately the same amount of artificial urine.

In addition, the absorbent cores of Example 1 and Example 2 exhibited excellent exudation resistance for absorbed liquids and also excellent form retention and thickness retention, compared to the absorbent core of Comparative Example 2 which had the upper layer as the gelling region (corresponding to the absorbent core disclosed in PTL 1).
The absorbent cores of Example 1 and Example 2 also exhibited equivalent absorption and more excellent exudation resistance, compared to Comparative Example 3 which had layers containing thickening agents for both the upper layer and lower layer.

### [Industrial Applicability]

The absorbent core of the invention can be used in sanitary products, such as sanitary napkins and panty liners, sanitary materials, such as disposable diapers, urine leakage-preventing sheets, urine-absorbing pads for incontinent patients, body fluid/blood-absorbing medical goods, wound-dressing materials, cosmetic pack materials, animal excrement-treating materials, agricultural and gardening products, freshness-keeping materials for foods, moisture condensation-proof materials and articles to be used in locations that require moisture absorption and/or moisture retention.

### [Explanation of Symbols]

1 Absorbent core
2 Upper layer
3 Lower layer
4 Gelling region
5 Thickening region
6 Front sheet
7 Rear sheet
8 Center region of lower layer
9 Peripheral region of lower layer
10 Absorbent article
11 Liquid-permeable top sheet.
12 Liquid-impermeable back sheet

## Claims

1. An absorbent core formed from at least two layers, **characterized in that** the absorbent core has in an upper layer a gelling region comprising a gelling agent that contains a polysaccharide capable of thickening in the presence of a polyvalent metal ion and a substance that can supply a polyvalent metal ion, and has in one or at least one of a plurality of lower layers, a thickening region comprising a thickening agent.

2. The absorbent core according to claim 1, wherein the polysaccharide capable of thickening in the presence of a polyvalent metal ion is selected from the group consisting of sodium alginate, propyleneglycol alginate, pectin, gellan gum, carrageenan, glucomannan and guar gum.

3. The absorbent core according to claim 1 or 2, wherein the substance that can supply a polyvalent metal ion is selected from the group consisting of calcium phosphate, calcium chloride, calcium lactate, calcium gluconate, calcium acetate, aluminum sulfate, aluminum nitrate, aluminum phosphate and aluminum acetate.

4. The absorbent core according to any one of claims 1 to 3, wherein the thickening agent is selected from the group consisting of sodium alginate, propyleneglycol alginate, pectin, gellan gum, carrageenan, glucomannan, guar gum, polyethylene oxide and polyethylene glycol.

5. The absorbent core according to any one of claims 1 to 4, which is composed of two layers, the upper layer and the lower layer.

6. The absorbent core according to any one of claims 1 to 5, wherein the gelling region further contains an organic acid and/or a polyvalent metal ion scavenger.

7. The absorbent core according to claim 6, wherein the organic acid is selected from the group consisting of glucono-δ-lactone, adipic acid, citric acid, malic acid, tartaric acid, lactic acid and acetic acid.

8. The absorbent core according to claim 6 or 7, wherein the polyvalent metal ion scavenger is selected from the group consisting of sodium citrate, sodium polyphosphate, sodium hexametaphosphate and sodium pyrophosphate.

9. The absorbent core according to any one of claims 1 to 8, wherein the thickening region contains a polyvalent metal ion scavenger.

10. An absorbent article comprising a liquid-permeable top sheet and a liquid-impermeable back sheet, and the absorbent core according to any one of claims 1 to 9 situated between the liquid-permeable top sheet and the liquid-impermeable back sheet.

11. The absorbent article according to claim 10, having water disintegratability.

12. The absorbent article according to claim 10 or 11, having biodegradability.
